# EUROPEAN PATENT APPLICATION

(11) **EP 4 840 947 A1**
(43) Date of publication of application: **18.11.2026**
(21) Application number: 25742309.5
(22) Date of filing: 16.01.2025
(51) Int. Cl.: C12N 9/00, C12N 15/70, A23K 10/16, A23K 20/189

(54) **NOVEL POLYPEPTIDE HAVING DON-DEGRADING ACTIVITY**

(30) Priority: 19.01.2024 KR 20240009010
(71) Applicant: CJ Cheiljedang Corporation, Seoul 04560 (KR)
(72) Inventor: KIM, Nam Yoon, Seoul 04560 (KR); LEE, Seung-hee, Seoul 04560 (KR); PARK, Taeseo, Seoul 04560 (KR); YANG, Tae Joo, Seoul 04560 (KR); KANG, Young Seo, Seoul 04560 (KR); LEE, Whiso, Seoul 04560 (KR)
(74) Representative: Meissner Bolte Partnerschaft mbB
(86) International application number: PCT/KR2025/099044
(87) International publication number: WO 2025/155161

(57) **Abstract**

Provided is a variant polypeptide having DON degradation activity.

## Description

### [Technical Field]

The present disclosure relates to a variant polypeptide having DON degradation activity.

### [Background Art]

Mycotoxins are a general term for metabolites derived from fungi, which are toxic to animals. When crops or stored agricultural products are infected or contaminated with fungi that produce mycotoxins, these mycotoxins are produced and pose a health risk to animals or humans. Trichothecenes, often found in grains, are sesquiterpenoid mycotoxins having a structure of 12,13-epoxi-trichothec-9-ene, and are produced primarily by *Fusarium*, *Myrothecium*, *Stachybotrys*, and *Trichothecium*. Among them, *Fusarium* is the most important trichothecene-producing species from an economic point of view, and among the toxins, the most commonly detected toxins in grains are deoxynivalenol (DON), nivalenol (NIV), and acetylated derivatives thereof.

Trichothecenes act on eukaryotic cells to exhibit various detrimental effects, such as inhibition of protein synthesis, cytotoxicity, cell death, etc. When feeds or foods contaminated with DON are consumed, people experience symptoms such as immunosuppression, anemia, headache, nausea, abdominal pain, etc., and animals experience symptoms such as refusal to eat, vomiting, growth retardation, reproductive disorders, etc. Due to global warming, it is expected that the annual exposure level of humans and animals to trichothecenes will exceed the tolerable level.

Most countries have laws regulating the allowable levels of DON in feed, foods, and harvested grains. Although attempts have been made to reduce DON contamination through pesticides targeting *Fusarium* spp. or breeding, DON still contaminates grains. In order to detoxify DON, various chemicals, such as ozone, ammonia, chlorine, hydrogen peroxide, sodium bisulfite, etc., have been treated, but they are not being scaled up due to cost, safety issues, negative effects on grain quality, etc. The most effective method is to treat with sodium metabisulfite. However, in Europe, this is prohibited for use in food grains, and accordingly, there is a need for a safe method of detoxifying DON.

### [Prior Art Documents]

### [Non-Patent Documents]

(Non-Patent Document 1) S Chakraborty et al, Virulence. 2015 Jan; 6(1): 50-65 (2014.12.17), Lactoylglutathione lyase, a critical enzyme in methylglyoxal detoxification, contributes to survival of Salmonella in the nutrient rich environment.

### [Disclosure]

### [Technical Problem]

A problem to be solved in the present disclosure is to provide a variant polypeptide having DON degradation activity.

### [Technical Solution]

An object of the present disclosure is to provide a variant polypeptide having deoxynivalenol (DON) degradation activity, wherein amino acids at positions corresponding to any one or more positions selected from positions 48, 60, 62, 83, and 133 of SEQ ID NO: 1 are substituted with different amino acids.

Another object of the present disclosure is to provide a polynucleotide encoding the variant polypeptide of the present disclosure.

Still another object of the present disclosure is to provide a microorganism comprising any one or more of the variant polypeptide; and the polynucleotide encoding the variant polypeptide.

Still another object of the present disclosure is to provide a composition for degrading deoxynivalenol (DON), the composition comprising any one or more of the variant polypeptide; and the microorganism expressing the variant polypeptide.

Still another object of the present disclosure is to provide a feed additive composition comprising any one or more of the variant polypeptide; and the microorganism expressing the variant polypeptide.

Still another object of the present disclosure is to provide a method of degrading DON, the method comprising the step of reacting DON with any one or more of the variant polypeptide; and the microorganism expressing the variant polypeptide.

Still another object of the present disclosure is to provide a method of preparing a feed product, the method comprising the step of mixing a feed component with the feed additive composition comprising any one or more of the variant polypeptide; and the microorganism expressing the variant polypeptide.

Still another object of the present disclosure is to provide a method of preparing the variant polypeptide having DON degradation activity, the method comprising the steps of:
culturing the microorganism comprising any one or more of the variant polypeptide; and the polynucleotide encoding the variant polypeptide; and
recovering the variant polypeptide having DON degradation activity of the present disclosure which is expressed in the culturing step.

### [Advantageous Effects]

A variant polypeptide having DON degradation activity of the present disclosure may be usefully applied to various industrial fields due to improved DON degradation activity.

### [Brief Description of the Drawing]

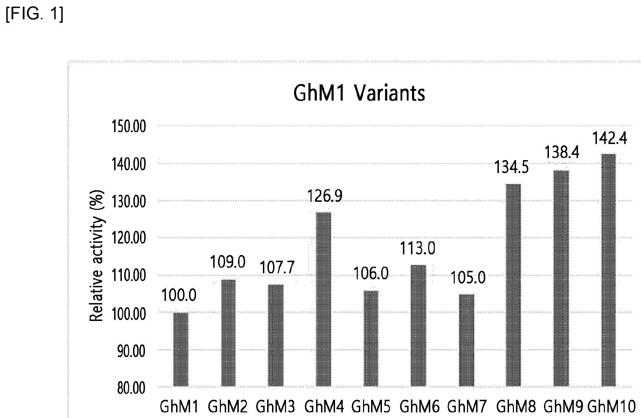

FIG. 1 shows the verification of the DON degradation activities of a parent DON degradation enzyme (GhM1: SEQ ID NO: 1) and variant polypeptides (GhM2 to GhM10) having DON degradation activity of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a variant polypeptide having deoxynivalenol (DON) degradation activity, wherein amino acids at positions corresponding to any one or more positions selected from positions 48, 60, 62, 83, and 133 of SEQ ID NO: 1 are substituted with different amino acids.

Further, the variant polypeptide may have substitutions of amino acids at positions corresponding to any one or more positions selected from positions 75 and 156 of SEQ ID NO: 1 with different amino acids, in addition to the substitutions of amino acids at positions corresponding to any one or more positions selected from positions 48, 60, 62, 83, and 133 of SEQ ID NO: 1.

As used herein, the term "deoxynivalenol (DON) degradation activity" means catalyzing the degradation of deoxynivalenol (DON) by converting the same into an iso-DON form.

In the present disclosure, deoxynivalenol (DON) has a chemical formula of C₁₅H₂₀O₆ and a molecular weight of 296.3 g/mol (CAS NO.: 51481-10-8), and may be a mycotoxin belonging to Type B trichothecene, which is one of the epoxy-sesquiterpenoids, and is also called vomitoxin. DON may be produced by fungi that cause diseases in plants, of which the genus *Fusarium* is a known producer, and when consumed, symptoms such as immunosuppression, anemia, headache, nausea, abdominal pain, etc. occur, and in animals, symptoms such as refusal to eat, vomiting, growth inhibition, and reproductive disorders, etc. may occur.

Meanwhile, the degradation of deoxynivalenol (DON) may be used in the same meaning as detoxification of DON, inactivation of DON, and removal of DON contamination (decontaminating).

Even though the variant polypeptide having DON degradation activity provided in the present disclosure is defined as a polypeptide having substitutions of amino acids at positions corresponding to any one or more positions selected from positions 48, 60, 62, 83, and 133 of SEQ ID NO: 1, or a polypeptide having substitutions of amino acids at positions corresponding to any one or more positions selected from positions 75 and 156 of SEQ ID NO: 1 with different amino acids, in addition thereto. However, the variant polypeptide does not exclude the addition of a meaningless sequence upstream or downstream the amino acid sequence of SEQ ID NO: 1, or a naturally occurring mutation, or a silent mutation thereof, and it is obvious to a person skilled in the art that those having activity identical or corresponding to that of the protein consisting of the amino acid sequence of SEQ ID NO: 1 fall within the scope of the polypeptide having DON degradation activity provided in the present disclosure.

In other words, although described as 'a protein or polypeptide having an amino acid sequence represented by a specific SEQ ID NO' or 'a protein or polypeptide comprising an amino acid sequence represented by a specific SEQ ID NO' in the present disclosure, it is obvious that any protein having an amino acid sequence with deletion, modification, substitution, or addition in part of the sequence may also be used in the present disclosure, as long as the protein may have an activity identical or corresponding to that of the polypeptide consisting of the amino acid sequence of the corresponding SEQ ID NO.

As used herein, the term "wild-type" refers to a naturally-occurring state without artificial modification. When the term "wild-type" is used in reference to a polypeptide, it refers to a naturally occurring polypeptide, which does not have artificial mutations (substitutions, insertions, deletions, etc.) in one or more amino acid positions. Similarly, when the term "wild-type" is used in reference to a polynucleotide, it means not having artificial modifications (substitutions, insertions, deletions) in one or more nucleotides. However, polynucleotides encoding wild-type polypeptides are not limited to wild-type polynucleotides, and also comprise sequences encoding any wild-type polypeptides.

As used herein, a parent sequence or backbone refers to a reference sequence in which modification is introduced to be a variant polypeptide. In other words, the parent sequence may be served as a starting sequence in which mutations such as substitutions, insertions, and/or deletions may be introduced. The parent sequence may be naturally occurring or wild-type, or a variant in which one or more substitutions, insertions, or deletions have occurred in the natural or wild-type, or may be an artificially synthesized sequence. When the parent sequence is an amino acid sequence exhibiting activity, i.e., an amino acid sequence of an enzyme, it may be referred to as a parent enzyme.

With respect to amino acid or nucleic acid sequences in the present disclosure, the term "fragment" means a part of a parent sequence. For example, it may be a polypeptide in the form in which one or more amino acids are removed from the C- or N-terminus of the parent sequence.

As used herein, the term "fragment" of an enzyme may refer to a "functional fragment". The "functional fragment" may also be referred to as an active fragment, and means a polypeptide that is a part of a parent enzyme and has the enzymatic activity of the parent enzyme. For example, the functional fragment of an enzyme may comprise a catalytic site of the enzyme.

The fragment of the enzyme may comprise a part of the full length of the parent enzyme. For example, the fragment of the enzyme may comprise amino acids of at least about 20%, 30%, 40%, 50%, 60%, 70%, 80%, 90%, 95%, 98%, 99% or more, or less than 100% of the full length of the parent enzyme, but is not limited thereto.

As used herein, the term "modifying" means changing or altering. This may be altering from a naturally occurring state. For example, an enzyme may be altered in such a way that the enzyme is altered from a parent or reference sequence.

In the present disclosure, a modified enzyme may be an enzyme that does not exist as it is in nature, i.e., a non-naturally occurring enzyme.

As used herein, the term "modified" means being altered, e.g., from its naturally occurring form. The modified enzyme of the present disclosure comprises non-naturally occurring enzymes or naturally occurring variants. For example, the modified enzyme of the present disclosure is a modified enzyme that has not been found in nature. For example, the modified enzyme of the present disclosure may be a non-spontaneously occurring enzyme, but is not limited thereto.

As used herein, the term "modification", when used with respect to amino acid/nucleic acid sequences, may comprise substitution of an amino acid/nucleic acid residue of a parent sequence for a different amino acid/nucleic acid residue in one or more positions in an amino acid sequence, deletion of an amino acid/nucleic acid residue (or series of amino acid/nucleic acid residues) of a parent sequence in one or more positions, insertion of an amino acid/nucleic acid residue (or series of amino acid/nucleic acid residues) of a parent sequence in one or more positions, truncation of the N-terminal and/or C-terminal amino acid sequences or 5' and/or 3' nucleic acid sequences, and any combination thereof.

As used herein, the term "variant" or "modified polypeptide" of an enzyme refers to a protein that has one or more amino acids different from the parent enzyme by conservative substitution and/or modification. The "variant" or "variant polypeptide" may be used interchangeably. The variant or variant polypeptide may be non-naturally occurring, but is not limited thereto.

The variant differs from the sequence of the parent enzyme by one or more modifications, e.g., substitutions, deletions, and/or insertions of amino acids.

Such variants may be generally identified by modifying one or more amino acids from the parent enzyme and evaluating the properties of the modified protein. That is, the ability of the variants may be enhanced, unchanged, or reduced, as compared to the parent enzyme.

Further, some variants may comprise variant polypeptides in which one or more regions, such as an N-terminal leader sequence or transmembrane domain, have been removed.

Other variants may comprise variants in which a part has been removed from the N- and/or C-terminal of a mature protein.

The term "variant" or "variant polypeptide" may be used interchangeably with terms such as modification, modified protein, mutant, mutein, divergent, variant, etc., and is not limited as long as the terms are used to indicate mutation.

The variants may comprise deletion or addition of amino acids that have minimal influence on the properties and secondary structure of a polypeptide. For example, the polypeptides may be conjugated with a signal (or leader) sequence at the N-terminal which is involved in the translocation of proteins co-translationally or post-translationally. Further, the polypeptides may also be conjugated with another sequence or linker to identify, purify, or synthesize the polypeptides.

As used herein, the term "conservative substitution" refers to the substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue.

As used herein, the "parent DON degradation enzyme" refers to a DON degradation enzyme that a modification is applied in order to produce the variant or the variant polypeptide of the present disclosure. Specifically, the parent DON degradation enzyme, parent enzyme, or parent sequence may be a naturally occurring polypeptide or a wild-type polypeptide, may be a mature polypeptide thereof, and may comprise a variant or functional fragment thereof, but is not limited thereto, as long as the polypeptide has DON degradation activity and may be a parent of the variant.

The parent DON degradation enzyme provided in the present disclosure may be, but is not limited to, a polypeptide of SEQ ID NO: 1. Further, it may be a polypeptide having about 80%, 85%, 90%, 95%, 96%, 97%, 98% or 99% or more sequence identity to the polypeptide of SEQ ID NO: 1 as long as it has DON degradation activity, and any polypeptide may be comprised within the scope of the parent DON degradation enzyme as long as it has the identical or corresponding activity to the polypeptide consisting of the amino acid sequence of SEQ ID NO: 1.

The parent DON degradation enzyme of the variant provided in the present disclosure may be derived from the genus *Gossypium*, the genus *Handroanthus*, or the genus *Hibiscus*. Specifically, it may be derived from the genus *Gossypium*.

In the present disclosure, the "variant polypeptide having DON degradation activity" may be a variant of the parent DON degradation enzyme.

As used herein, the term "variant of the parent DON degradation enzyme" or "variant polypeptide having DON degradation activity" refers to a protein having DON degradation activity, in which one or more amino acids are different from the amino acid sequence of the parent DON degradation enzyme.

The "variant polypeptide having DON degradation activity", the "variant of the parent DON degradation enzyme", and the "DON degradation enzyme variant" may be used interchangeably with each other.

The variant polypeptide provided in the present disclosure may comprise modification of one or more amino acids in the sequence of the parent DON degradation enzyme, while having DON degradation activity. The modification may be the substitution of amino acids and/or formation of disulfide bonds.

Specifically, the variant provided in the present disclosure may have one or more altered functions or properties compared to the parent DON degradation enzyme by comprising the modification of one or more amino acids in the sequence of the parent DON degradation enzyme, while having DON degradation activity.

In one specific embodiment, the variant provided in the present disclosure may have one or more altered functions or properties compared to the parent DON degradation enzyme by comprising modification of one or more amino acids in the sequence of the parent DON degradation enzyme, and may have one or more conservative substitution, while having DON degradation activity.

The variant provided in the present disclosure, which is a variant of the parent DON degradation enzyme, may be a polypeptide having DON degradation activity. In one specific embodiment, the variant provided in the present disclosure may comprise the modification at positions corresponding to any one or more positions selected from positions 48, 60, 62, 83, and 133 of SEQ ID NO: 1.

In another specific embodiment, the variant provided in the present disclosure may comprise modifications at positions corresponding to any one or more positions selected from positions 75 and 156 of SEQ ID NO: 1, in addition to substitutions of amino acids at positions corresponding to any one or more positions selected from positions 48, 60, 62, 83, and 133 of SEQ ID NO: 1.

In the present disclosure, the position number is a position corresponding to the position of the polypeptide of SEQ ID NO: 1, and the term "corresponding" is as described above.

In one specific embodiment, the variant polypeptide of the present disclosure may comprise substitutions of amino acids at positions corresponding to any one or more positions selected from positions 48, 60, 62, 83, and 133 of SEQ ID NO: 1, as compared to the amino acid sequence of SEQ ID NO: 1, but is not limited thereto. Further, the variant polypeptide of the present disclosure may comprise substitutions at positions corresponding to any one or more positions selected from positions 75 and 156 of SEQ ID NO: 1, in addition thereto.

For a specific example thereof, the variant polypeptide provided in the present disclosure may comprise substitutions of combinations of 2, 3, 4, 5, 6, or 7 positions selected from the positions with different amino acids.

In any one specific embodiment of the above-described specific embodiments, the variant polypeptide provided in the present disclosure may comprise substitutions of amino acids at positions corresponding to any one or more positions selected from positions 48, 60, 62, 83, and 133 of SEQ ID NO: 1 with G, A, V, L, I, M, F, W, P, S, T, C, Y, N, Q, D, E, K, R, or H.

In any one specific embodiment of the above-described specific embodiments, the variant polypeptide provided in the present disclosure may comprise substitutions of amino acids at positions corresponding to any one or more positions selected from positions 48, 60, 62, 83, and 133 of SEQ ID NO: 1 with polar, nonpolar, hydrophobic, or acidic amino acids.

In any one specific embodiment of the above-described specific embodiments, the variant polypeptide provided in the present disclosure may comprise a substitution of the amino acid corresponding to position 48 of SEQ ID NO: 1 with glutamic acid; a substitution of the amino acid corresponding to position 60 of SEQ ID NO: 1 with isoleucine; a substitution of the amino acid corresponding to position 62 of SEQ ID NO: 1 with glutamic acid or glutamine; a substitution of the amino acid corresponding to position 83 of SEQ ID NO: 1 with valine; or a substitution of the amino acid corresponding to position 133 of SEQ ID NO: 1 with valine, but is not limited thereto.

Further, in another specific embodiment of the above-described specific embodiments, the variant polypeptide provided in the present disclosure may comprise substitutions of amino acids at positions corresponding to any one or more positions selected from positions 75 and 156 of SEQ ID NO: 1 with different amino acids, in addition to the substitutions of amino acids at positions corresponding to any one or more positions selected from positions 48, 60, 62, 83, and 133 of SEQ ID NO: 1.

In any one specific embodiment, the variant polypeptide provided in the present disclosure may further comprise substitutions of amino acids at positions corresponding to any one or more positions selected from positions 75 and 156 of SEQ ID NO: 1 with polar, nonpolar, hydrophobic, or acidic amino acids.

In another specific embodiment of the above-described specific embodiments, the variant polypeptide provided in the present disclosure may further comprise a substitution of the amino acid corresponding to position 75 of SEQ ID NO: 1 with leucine; or a substitution of the amino acid corresponding to position 156 of SEQ ID NO: 1 with valine; or a combination thereof.

In still another specific embodiment, the variant polypeptide provided in the present disclosure may comprise a substitution of the amino acid corresponding to position 60 of SEQ ID NO: 1 with isoleucine; and a substitution of the amino acid corresponding to position 75 of SEQ ID NO: 1 with leucine.

In still another specific embodiment, the variant polypeptide provided in the present disclosure may comprise a substitution of the amino acid corresponding to position 60 of SEQ ID NO: 1 with isoleucine; and a substitution of the amino acid corresponding to position 156 of SEQ ID NO: 1 with valine.

In still another specific embodiment, the variant polypeptide provided in the present disclosure may comprise a substitution of the amino acid corresponding to position 60 of SEQ ID NO: 1 with isoleucine; a substitution of the amino acid corresponding to position 75 of SEQ ID NO: 1 with leucine; and a substitution of the amino acid corresponding to position 156 of SEQ ID NO: 1 with valine.

As used herein, the term "corresponding to" refers to an amino acid residue at the position recited in a protein or polypeptide, or an amino acid residue that is similar, identical, or homologous to the residue recited in a protein or polypeptide. Identifying an amino acid at a corresponding position may mean determining a particular amino acid in a sequence that refers to a particular sequence. As used herein, the "corresponding region" generally refers to a similar or corresponding position in a related protein or reference protein.

In the present disclosure, SEQ ID NO: 1 may be used as a reference sequence to determine the position of amino acids in any amino acid sequence.

As used herein, the term "reference sequence" means a sequence used to determine the position of amino acids within any amino acid sequence. Any amino acid sequence may be aligned with a reference sequence to determine the position of an amino acid that corresponds to a particular position in the reference sequence within any amino acid sequence.

That is, SEQ ID NO: 1 disclosed herein may be used to determine the corresponding amino acid residues in any polypeptide having DON degradation activity. Unless indicated otherwise in the present disclosure, residues of a particular amino acid sequence are numbered based on SEQ ID NO: 1.

For example, any amino acid sequence is aligned with SEQ ID NO: 1, and based on the alignment, each amino acid residue of the amino acid sequence may be numbered with reference to the numerical position of the amino acid residue corresponding to the amino acid residue of SEQ ID NO: 1. For example, a sequence alignment algorithm such as that described herein may identify the position of an amino acid or a position where modifications such as substitutions, insertions or deletions occur, compared to a query sequence (also referred to as a "reference sequence").

In such alignment, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), etc. may be used, but are not limited thereto.

Further, the corresponding amino acid residue in another polypeptide having DON degradation activity may be identified by multiple sequence alignment. Examples of multiple sequence alignment known in the art comprise programs such as MUSCLE (multiple sequence comparison by log-expectation; 3.5 or later; Edgar, 2004, Nucleic Acids Research 32: 1792-1797), MAFFT (6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22 : 4673-4680), etc., and their respective default parameters may be used, but are not limited thereto.

In addition, when enzymes that have diverged from the mature polypeptide of SEQ ID NO: 1 fail to show their relationship by traditional sequence-based comparison, other pairwise sequence comparison algorithms may be used (Lindahl and Elofsson, 2000, J. Mol. Biol. 295: 613-615). Higher sensitivity in sequence-based searching may be attained using search programs that utilize probabilistic representations of polypeptide families (profiles) to search databases. For example, the PSI BLAST program generates profiles through an iterative database search process and is capable of detecting remote homologs (Atschul et al., 1997, Nucleic Acids Res. 25: 3389-3402). Even greater sensitivity may be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be used in sequence to generate homology models for the polypeptides, and such models may be assessed for accuracy using a variety of tools developed for that purpose.

For proteins of known structure, several tools and resources are available for retrieving and generating structural alignments. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or CE (Combinatorial extension) Shindyalov and Bourne, 1998, Protein Engineering 11: 739-747). Implementation of these algorithms may be additionally utilized to query structure databases with a structure of interest in order to discover possible structural homologs (Holm and Park, 2000, Bioinformatics 16: 566-567).

The above methods are illustrative, and are not limited thereto.

Throughout the entire specification of the present disclosure, the conventional one-letter and three-letter codes for naturally occurring amino acids are used. Further, the amino acids abbreviated herein are described according to the nomenclature rules of IUPAC-IUB.

| | |
|---|---|
| alanineAla, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Meanwhile, any amino acid may be described as Xaa or X.

Further, three-letter codes are generally allowed for not only naturally occurring amino acids, but also for other amino acids, such as 2-Aminoisobutyric acid (Aib), Sar (N-methylglycine), α-methyl-glutamic acid, etc., which be used.

Amino acids may be generally classified based on similarity of polarity, charge, solubility, hydrophobicity, hydrophilicity, and/or amphipathic nature of a residue. Accordingly, amino acid substitution may generally occur based on similarity of polarity, charge, solubility, hydrophobicity, hydrophilicity, and/or amphipathic nature of a residue.

For example, among the amino acids having an electrically charged side chain (electrically charged amino acid), positively charged (basic) amino acids comprise arginine, lysine, and histidine; negatively charged (acidic) amino acids comprise glutamic acid and aspartic acid; among the amino acids having an uncharged side chain (uncharged amino acid), nonpolar amino acids comprise glycine, alanine, valine, leucine, isoleucine, methionine, phenylalanine, tryptophan, and proline; polar or hydrophilic amino acids comprise serine, threonine, cysteine, tyrosine, asparagine, and glutamine; and among the nonpolar amino acids, aromatic amino acids comprise phenylalanine, tryptophan, and tyrosine.

In one embodiment, the variant polypeptide having DON degradation activity provided in the present disclosure may comprise a variant polypeptide, in which amino acids at positions corresponding to any one or more positions selected from positions 48, 60, 62, 83, and 133 in SEQ ID NO: 1 are substituted, or may consist of SEQ ID NO: 3, SEQ ID NO: 5, SEQ ID NO: 7, SEQ ID NO: 9, SEQ ID NO: 11, or SEQ ID NO: 13. In another embodiment, the novel variant polypeptide having DON degradation activity provided in the present disclosure may have at least 80%, 90%, 91%, 92%, 93%, 94%, 95%, 96%, 97%, 98%, 99% or more, or 100% homology or identity to SEQ ID NO: 3, SEQ ID NO: 5, SEQ ID NO: 7, SEQ ID NO: 9, SEQ ID NO: 11, and SEQ ID NO: 13, or may comprise a sequence having the homology or identity, or may essentially consist of or may consist of the same.

In another embodiment, the variant polypeptide having DON degradation activity provided in the present disclosure may comprise a variant polypeptide having substitutions of amino acids at positions corresponding to any one or more positions selected from positions 75 and 156 in SEQ ID NO: 1, in addition to substitutions of amino acids at positions corresponding to any one or more positions selected from positions 48, 60, 62, 83, and 133 of SEQ ID NO: 1, or may consist of SEQ ID NO: 15, SEQ ID NO: 17, or SEQ ID NO: 19. In another embodiment, the novel polypeptide provided in the present disclosure may have at least 80%, 90%, 91%, 92%, 93%, 94%, 95%, 96%, 97%, 98%, 99% or more, or 100% homology or identity to SEQ ID NO: 15, SEQ ID NO: 17, or SEQ ID NO: 19, or may comprise a sequence having the homology or identity, or may essentially consist of or may consist of the same.

As used herein, the term 'homology' or 'identity' refers to a degree of relevance between two given amino acid sequences or nucleotide sequences, and may be expressed as a percentage. The terms 'homology and identity' may often be used interchangeably with each other.

The sequence homology or identity of conserved polynucleotides or polypeptides may be determined by standard alignment algorithms and may be used with a default gap penalty established by the program being used. Substantially, homologous or identical sequences may generally hybridize under moderately or highly stringent conditions to the entire length of the sequence or at least about 50%, 60%, 70%, 80%, or 90% of the full-length. It is apparent that hybridization also comprises hybridization of a polynucleotide with a polynucleotide comprising a general codon or a codon in consideration of codon degeneracy.

Whether any two polynucleotide or polypeptide sequences have homology, similarity, or identity may be, for example, determined using a known computer algorithm such as the "FASTA" program using default parameters, as in Pearson et al (1988) [Proc. Natl. Acad. Sci. USA 85]: 2444. Alternatively, it may be determined by the Needleman Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073. For example, the homology, similarity, or identity may be determined using BLAST or ClustalW of the National Center for Biotechnology Information.

The homology, similarity, or identity of polynucleotides or polypeptides may be, for example, determined by comparing sequence information using, for example, the GAP computer program, such as Needleman et al. (1970), J. Mol. Biol. 48: 443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of symbols in the shorter of the two sequences. Default parameters for the GAP program may comprise (1) a binary comparison matrix (comprising a value of 1 for identities and 0 for non-identities) and the weighted comparison matrix of Gribskov et al., (1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York).

As used herein, "enzymatic activity" or "DON degradation activity" refers to at least one catalytic activity. Specifically, it may be the conversion efficiency of an enzyme, which is mainly expressed as kcat/Km, but is not limited thereto.

When the enzyme is completely saturated with substrates, kcat refers to the rate constant (catalytic constant) that converts substrates into products in a unit time by one enzyme, and is also referred to as a turnover number. Km is the substrate concentration when the reaction rate is at half the maximum value (Vmax).

Examples of the ways to express enzymatic activity comprise specific activity (umol of converted substrate x mg ⁻¹ x min ⁻¹) or volumetric activity (umol of converted substrate x mL ⁻¹ x min ⁻¹), etc. However, defining the enzymatic activity is not limited to the description described above, and the enzymatic activity may be defined and evaluated, based on the information known in the art.

As used herein, "enzyme stability" means that enzymatic activity is maintained during storage or reaction time. In order to measure such changes in stability, the initial enzymatic activity may be measured and compared under defined conditions at time zero (100%) and after a predetermined period of time (x%), and therefore, the level at which the enzymatic activity is lost or the enzyme stability may be expressed.

As used herein, the term "specific activity" refers to the activity of an enzyme per unit weight of proteins, and may be expressed as unit/mg. The quantification of proteins may be performed using, for example, SDS-PAGE or Bradford assay, etc.

Factors that affect enzymatic activity comprise, for example, pH, heat, the presence of other substances (e.g., oxidizing agents, chelating agents), etc.

In one specific embodiment, the variant polypeptide provided in the present disclosure may have an increased enzymatic activity of about 100%, about 101%, about 102%, about 103%, about 104%, about 105%, about 106%, about 107%, about 108%, about 109%, about 110%, about 111%, about 112%, about 113%, about 114%, about 115%, about 116%, about 117%, about 118%, about 119%, about 120%, about 121%, about 122%, about 123%, about 124%, about 125%, about 126%, about 127%, about 128%, about 129%, about 130%, about 131%, about 132%, about 133%, about 134%, about 135%, about 136%, about 137%, about 138%, about 139%, about 140%, about 141%, about 142%, about 145% or about 150% or more, as compared to the parent enzyme.

In any one embodiment of the above-described embodiments, the variant polypeptide having increased enzymatic activity (improved DON degradation activity), as compared to the polypeptide of SEQ ID NO. 1, among the variant polypeptides, may comprise any one or more substitutions selected from the following substitutions.

| |
|---|
| R48E |
| V60I |
| D62E |
| D62Q |
| V75L |
| T133V |
| A156V |
| I83V |
| R48E, V60I |
| R48E, D62E or D62Q |
| R48E, V75L |
| R48E, T133V |
| R48E, A156V |
| R48E, I83V |
| V60I, D62E or D62Q |
| V60I, V75L |
| V60I, T133V |
| V60I, A156V |
| V60I, I83V |
| D62E or D62Q, V75L |
| D62E or D62Q, T133V |
| D62E or D62Q, A156V |
| D62E or D62Q, I83V |
| V75L, T133V |
| V75L, A156V |
| V75L, I83V |
| T133V, A156V |
| T133V, I83V |
| A156V, I83V |
| R48E, V60I, D62E or D62Q |
| R48E, V60I, V75L |
| R48E, V60I, T133V |
| R48E, V60I, A156V |
| R48E, V60I, I83V |
| R48E, D62E or D62Q, V75L |
| R48E, D62E or D62Q, T133V |
| R48E, D62E or D62Q, A156V |
| R48E, D62E or D62Q, I83V |
| R48E, V75L, T133V |
| R48E, V75L, A156V |
| R48E, V75L, I83V |
| R48E, T133V, A156V |
| R48E, T133V, I83V |
| R48E, A156V, I83V |
| V60I, D62E or D62Q, V75L |
| V60I, D62E or D62Q, T133V |
| V60I, D62E or D62Q, A156V |
| V60I, D62E or D62Q, I83V |
| V60I, V75L, T133V |
| V60I, V75L, A156V |
| V60I, V75L, I83V |
| V60I, T133V, A156V |
| V60I, T133V, I83V |
| V60I, A156V, I83V |
| D62E or D62Q , V75L, T133V |
| D62E or D62Q , V75L, A156V |
| D62E or D62Q , V75L, I83V |
| D62E or D62Q , T133V, A156V |
| D62E or D62Q , T133V, I83V |
| D62E or D62Q , A156V, I83V |
| V75L, T133V, A156V |
| V75L, T133V, I83V |
| V75L, A156V, I83V |
| T133V, A156V, I83V |
| R48E, V60I, D62E or D62Q, V75L |
| R48E, V60I, D62E or D62Q, T133V |
| R48E, V60I, D62E or D62Q, A156V |
| R48E, V60I, D62E or D62Q, I83V |
| R48E, V60I, V75L, T133V |
| R48E, V60I, V75L, A156V |
| R48E, V60I, V75L, I83V |
| R48E, V60I, T133V, A156V |
| R48E, V60I, T133V, I83V |
| R48E, V60I, A156V, I83V |
| R48E, D62E or D62Q, V75L, T133V |
| R48E, D62E or D62Q, V75L, A156V |
| R48E, D62E or D62Q, V75L, I83V |
| R48E, D62E or D62Q, T133V, A156V |
| R48E, D62E or D62Q, T133V, I83V |
| R48E, D62E or D62Q, A156V, I83V |
| R48E, V75L, T133V, A156V |
| R48E, V75L, T133V, I83V |
| R48E, V75L, A156V, I83V |
| R48E, T133V, A156V, I83V |
| V60I, D62E or D62Q, V75L, T133V |
| V60I, D62E or D62Q, V75L, A156V |
| V60I, D62E or D62Q, V75L, I83V |
| V60I, D62E or D62Q, T133V, A156V |
| V60I, D62E or D62Q, T133V, I83V |
| V60I, D62E or D62Q, A156V, I83V |
| V60I, V75L, T133V, A156V |
| V60I, V75L, T133V, I83V |
| V60I, V75L, A156V, I83V |
| V60I, T133V, A156V, I83V |
| D62E or D62Q, V75L, T133V, A156V |
| D62E or D62Q, V75L, T133V, I83V |
| D62E or D62Q, V75L, A156V, I83V |
| D62E or D62Q, T133V, A156V, I83V |
| V75L, T133V, A156V, I83V |
| R48E, V60I, D62E or D62Q, V75L, T133V |
| R48E, V60I, D62E or D62Q, V75L, A156V |
| R48E, V60I, D62E or D62Q, V75L, I83V |
| R48E, V60I, D62E or D62Q, T133V, A156V |
| R48E, V60I, D62E or D62Q, T133V, I83V |
| R48E, V60I, D62E or D62Q, A156V, I83V |
| R48E, V60I, V75L, T133V, A156V |
| R48E, V60I, V75L, T133V, I83V |
| R48E, V60I, V75L, A156V, I83V |
| R48E, V60I, T133V, A156V, I83V |
| R48E, D62E or D62Q, V75L, T133V, A156V |
| R48E, D62E or D62Q, V75L, T133V, I83V |
| R48E, D62E or D62Q, V75L, A156V, I83V |
| R48E, D62E or D62Q, T133V, A156V, I83V |
| R48E, V75L, T133V, A156V, I83V |
| V60I, D62E or D62Q, V75L, T133V, A156V |
| V60I, D62E or D62Q, V75L, T133V, I83V |
| V60I, D62E or D62Q, V75L, A156V, I83V |
| V60I, D62E or D62Q, T133V, A156V, I83V |
| V60I, V75L, T133V, A156V, I83V |
| D62E or D62Q, V75L, T133V, A156V, I83V |
| R48E, V60I, D62E or D62Q, V75L, T133V, A156V |
| R48E, V60I, D62E or D62Q, V75L, T133V, I83V |
| R48E, V60I, D62E or D62Q, V75L, A156V, I83V |
| R48E, V60I, D62E or D62Q, T133V, A156V, I83V |
| R48E, V60I, V75L, T133V, A156V, I83V |
| R48E, D62E or D62Q, V75L, T133V, A156V, I83V |
| V60I, D62E or D62Q, V75L, T133V, A156V, I83V |
| R48E, V60I, D62E or D62Q, V75L, T133V, A156V, I83V |

In any one embodiment of the above-described embodiments, the properties possessed by the variant polypeptide having DON degradation activity, which is provided in the present disclosure, may be suitable or improved activity for application in various industrial fields, comprising feeds, baking, pulp bleaching, etc.

Another aspect of the present disclosure provides a polynucleotide encoding the variant polypeptide having DON degradation activity of the present disclosure.

As used herein, the term "polynucleotide", which is a long chain polymer of nucleotides formed by linking nucleotide monomers via covalent bonds, refers to a DNA or RNA strand having a predetermined length or more.

The polynucleotide encoding the variant polypeptide having DON degradation activity of the present disclosure may comprise any polynucleotide without limitation, as long as it is a polynucleotide encoding the variant polypeptide; the polypeptide of SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17 or SEQ ID NO: 19, or the polypeptide having activity corresponding thereto.

The polynucleotide encoding the variant polypeptide having DON degradation activity of the present disclosure may undergo various modifications in the coding region within a scope that does not change the amino acid sequence of the polypeptide, due to codon degeneracy or in consideration of the codons preferred in an organism in which the variant polypeptide is to be expressed.

For example, the polynucleotide encoding the variant polypeptide having DON degradation activity of the present disclosure may be a polynucleotide sequence encoding an amino acid sequence of SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17 or SEQ ID NO: 19, or a polypeptide having homology or identity thereto.

In one embodiment, the polynucleotide encoding the variant polypeptide having DON degradation activity of the present disclosure may have or comprise a nucleotide sequence having at least 50%, 60%, 70%, 75%, 80%, 85% or 90% or more, and less than 100% homology or identity to a polynucleotide sequence of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, or SEQ ID NO: 20. In another embodiment, the polynucleotide encoding the variant polypeptide having DON degradation activity of the present disclosure may consist of or essentially consist of a nucleotide sequence having at least 70%, 75%, 80%, 85%, 90%, 95%, 96%, 97%, 98%, or 99% or more homology or identity to the sequence of SEQ ID NO: SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, or SEQ ID NO: 20, but is not limited thereto. Further, the polynucleotide of the present disclosure may comprise a probe that may be prepared from a known gene sequence, e.g., any sequence capable of hybridizing with a sequence complementary to all or part of the polynucleotide sequence of the present disclosure under stringent conditions, without limitation.

The "stringent conditions" refers to conditions under which specific hybridization between polynucleotides is allowed. Such conditions are specifically described in the literature (see J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may comprise conditions under which polynucleotides having high homology or identity, polynucleotides having 70% or more, 75% or more, 80% or more, 85% or more, 90% or more, 95% or more, 96% or more, 97% or more, 98% or more, or 99% or more homology or identity are hybridized with each other, and polynucleotides having homology or identity lower than the above are not hybridized with each other, or washing conditions of the common Southern hybridization, i.e., washing once, specifically, twice or three times at a salt concentration and a temperature corresponding to 60°C, 1×SSC, 0.1% SDS, specifically, 60°C, 0.1×SSC, 0.1% SDS, and more specifically 68°C, 0.1×SSC, 0.1% SDS.

Hybridization requires that two nucleic acids comprise complementary sequences, although mismatches between bases are possible depending on the stringency of the hybridization. The term "complementary" is used to describe the relationship between nucleotide bases that may hybridize with each other. For example, with respect to DNA, adenine is complementary to thymine, and cytosine is complementary to guanine. Therefore, the polynucleotide of the present disclosure may comprise isolated nucleotide fragments complementary to the entire sequence as well as nucleic acid sequences substantially similar thereto.

Specifically, polynucleotides having homology or identity to the polynucleotide of the present disclosure may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (e.g., J. Sambrook et al., supra).

Still another aspect of the present disclosure provides a vector comprising the polynucleotide encoding the variant polypeptide having DON degradation activity of the present disclosure. The variant polypeptide and polynucleotide are as described in other aspects.

As used herein, the term "vector" refers to a DNA construct comprising the nucleotide sequence of the polynucleotide encoding the desired polypeptide operably linked to a suitable expression regulatory region (expression regulatory sequence) so as to express the desired polypeptide in a suitable host cell. The expression regulatory region may comprise a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

For example, the polynucleotide encoding the desired protein may be replaced with a mutated polynucleotide in the chromosome through a vector for intracellular chromosomal insertion. The insertion of the polynucleotide into the chromosome may be performed by any method known in the art, e.g., by homologous recombination, but is not limited thereto. The vector may further comprise a selection marker to confirm the insertion into the chromosome. The selection marker is for selecting the cells transformed with the vector, i.e., for confirming whether the desired nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or exhibit different phenotypes under the environment treated with the selective agent, and thus the transformed cells may be selected.

The vector used in the present disclosure is not particularly limited, and any vector known in the art may be used.

Examples of the vectors commonly used in prokaryotic cells may comprise, as phage vectors or cosmid vectors, pWE15, M13, MBL3, MBL4, IXII, ASHII, APII, t10, t11, Charon4A, Charon21A, etc.; and as plasmid vectors, those based on pBR, pUC, pBluescriptII, pGEM, pTZ, pCL and pET, etc. Specifically, pDZ, pACYC177, pACYC184, pCL, pECCG117, pUC19, pBR322, pMW118, pCC1BAC, pDCM2 vector, etc. may be used.

Examples of the vectors used in eukaryotic cells may comprise, as yeast expression vectors, integrative yeast plasmids (YIp) and extrachromosomal plasmid vectors. The extrachromosomal plasmid vectors may comprise episomal yeast plasmids (YEp), replicative yeast plasmids (YRp), and yeast centromeric plasmids (YCp). Further, artificial yeast chromosomes (YACs) may also be used as the vector of the present disclosure. Specific examples of the available vectors may comprise pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405 and pYJ406, but are not limited thereto.

As used herein, the term "transformation" means that a vector comprising a polynucleotide encoding a target protein is introduced into a host cell or a microorganism so that the protein encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and RNA encoding a desired protein. The polynucleotide may be introduced in any form as long as it may be introduced into a host cell and expressed. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the gene sequence is functionally linked to a promoter sequence that initiates and mediates transcription of the polynucleotide encoding the desired polypeptide of the present disclosure.

The method of transforming the vector of the present disclosure comprises any method of introducing the nucleic acid into a cell, and may be performed by selecting a suitable standard technique known in the art according to the host cell. For example, the transformation method may comprise electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, etc., but is not limited thereto.

Still another aspect of the present disclosure provides a microorganism comprising any one or more of the variant polypeptide having DON degradation activity of the present disclosure; and the polynucleotide encoding the variant polypeptide.

The microorganism may comprise the above-described variant polypeptide, the polynucleotide encoding the same, a nucleic construct and/or vector comprising the polynucleotide. The nucleic acid construct or vector may be integrated into the chromosome, or may remain as a self-replicating extrachromosomal vector.

The microorganism of the present disclosure may comprise any microorganism without limitation as long as it is able to express the variant polypeptide having DON degradation activity of the present disclosure. For example, the microorganism may comprise any cell useful in the recombinant production of the variant polypeptide having DON degradation activity of the present disclosure. For example, the microorganism may be a prokaryotic or eukaryotic cell. For another example, examples of the microorganism may comprise fungi and bacteria.

In one embodiment, the microorganism of the present disclosure may be a microorganism of the genus *Escherichia*, and may be *Escherichia coli* (*E.coli*), *Escherichia albertii*, *Escherichia fergusonii*, *Escherichia hermannii*, *Escherichia vulneris*, or *Escherichia blattae*. In any one embodiment of the above-described embodiments, the microorganism may be *Escherichia coli*, but is not limited thereto.

Still another aspect of the present disclosure provides a composition for degrading deoxynivalenol (DON), the composition comprising any one or more of the variant polypeptide having DON degradation activity of the present disclosure; and the microorganism expressing the variant polypeptide.

Still another aspect of the present disclosure provides a method of degrading DON, the method comprising the step of reacting DON with any one or more of the variant polypeptide having DON degradation activity of the present disclosure; and the microorganism expressing the variant polypeptide.

The variant polypeptide having DON degradation activity of the present disclosure and/or the microorganism expressing the variant polypeptide may be used in degrading substances comprising DON.

In one specific embodiment, the polypeptide of the present disclosure may be used in degradation of DON toxin, detoxification of DON, conversion of DON (deoxynivalenol) to iso-DON (iso-deoxynivalenol), and/or the induction of degradation through the conversion of iso-DON.

Further, in another additional embodiment of the present disclosure, the polypeptide of the present disclosure may be used in detoxification of foods contaminated with DON, removal of contamination (decontaminating), or detoxification (e.g., degradation).

In one embodiment, the composition for degrading DON provided in the present disclosure may further comprise a naturally occurring substance or non-naturally occurring substance, in addition to the variant polypeptide of the present disclosure.

In one embodiment, the composition for degrading DON provided in the present disclosure may further comprise any components suitable for application to various industrial fields such as animal feeds, baking, biomass saccharification, pulp bleaching, etc.

Examples of the substances that may be added comprise stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, etc., but are not limited thereto.

Still another aspect of the present disclosure provides a feed additive composition comprising any one or more of the variant polypeptide having DON degradation activity of the present disclosure; and the microorganism expressing the variant polypeptide.

Still another aspect of the present disclosure provides a method of preparing a feed product, the method comprising the step of mixing a feed component with the feed additive composition comprising any one or more of the variant polypeptide having DON degradation activity of the present disclosure; and the microorganism expressing the variant polypeptide.

The variant polypeptide having DON degradation activity of the present disclosure and/or the microorganism expressing the variant polypeptide may be used in any one of the following applications:
a) an additive in animal feedstuffs; and/or
b) a feed supplement for animals; and/or
c) breakdown of grain-based materials (e.g., this may be whole grain or part of grain).

The feed composition of the present disclosure may refer to any natural or artificial diet, a single meal, etc., or a component of the single meal, which is eaten, ingested, and digested by an animal, or which is suitable therefor, and the feed composition may be prepared in various forms known in the art.

In one embodiment, the feed product may be a grain-based material (comprising whole or part of grains, or malted grains, e.g., wheat, barley, corn, oats, rye, rice, sorghum, etc.).

Still another aspect of the present disclosure provides a method of preparing the variant polypeptide having DON degradation activity, the method comprising the steps of:
culturing the microorganism comprising any one or more of the variant polypeptide having DON degradation activity of the present disclosure; and the polynucleotide encoding the variant polypeptide; and
recovering the variant polypeptide having DON degradation activity of the present disclosure, which is expressed in the culturing step.

The culturing may comprise the step of culturing the microorganism in a culture medium.

As used herein, the term "culturing" means that the host cell is grown under appropriately controlled environmental conditions. The culturing process of the present disclosure may be performed in a suitable medium and culture conditions known in the art. Such a culturing process may be easily adjusted for use by those skilled in the art according to the strain to be selected. Specifically, the culturing may be a batch culture, a continuous culture, and a fed-batch culture, but is not limited thereto.

As used herein, the term "medium" refers to a mixture of materials which comprise nutrient materials required for culturing the host cell as a main ingredient, and it supplies nutrient materials and growth factors, along with water that is essential for survival and growth. Specifically, the medium and other culture conditions used for culturing the host cell of the present disclosure may comprise any medium commonly used for culturing host cells without any particular limitation. However, the host cell of the present disclosure may be cultured under aerobic conditions in a common medium comprising appropriate carbon sources, nitrogen sources, phosphorus sources, inorganic compounds, amino acids, and/or vitamins, while adjusting temperature, pH, etc.

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc. Further, natural organic nutrients such as starch hydrolysate, molasses, blackstrap molasses, rice bran, cassava, sugar cane molasses, and corn steep liquor, etc. may be used. Specifically, carbohydrates such as glucose and sterilized pretreated molasses (i.e., molasses converted to reducing sugar) may be used, and in addition, various other carbon sources may be used in an appropriate amount without limitation. These carbon sources may be used alone or in a combination of two or more thereof, but are not limited thereto.

The nitrogen source may comprise inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extract, yeast extract, malt extract, corn steep liquor, casein hydrolysate, fish or decomposition product thereof, defatted soybean cake or decomposition product thereof, etc. These nitrogen sources may be used alone or in a combination of two or more thereof, but are not limited thereto.

The phosphorus source may comprise monopotassium phosphate, dipotassium phosphate, or corresponding sodium-comprising salts, etc. Examples of the inorganic compounds may comprise sodium chloride, calcium chloride, iron chloride, magnesium sulfate, iron sulfate, manganese sulfate, calcium carbonate, etc. In addition, amino acids, vitamins, and/or appropriate precursors may be comprised. These components or precursors may be added to a medium in a batch or continuous manner, but are not limited thereto.

Further, the pH of the medium may be adjusted by adding a compound such as ammonium hydroxide, potassium hydroxide, ammonia, phosphoric acid, sulfuric acid, etc. during the culturing of the host cell in an appropriate manner. In addition, foam formation may be prevented during the culturing by using an antifoaming agent such as fatty acid polyglycol ester. Further, oxygen or oxygen-comprising gas may be injected to the medium in order to maintain aerobic conditions of the medium; or no gas may be injected, or nitrogen, hydrogen, or carbon dioxide gas may be injected in order to maintain anaerobic or microaerobic conditions, but is not limited thereto.

The temperature of the medium may be 20°C to 55°C, specifically, 25°C to 40°C, but is not limited thereto. The culturing may be continued until the desired amount of a useful material is obtained, and may be specifically carried out for 24 hours to 196 hours, but is not limited thereto.

In one embodiment, the method of preparing the variant polypeptide having DON degradation activity of the present disclosure may further comprise the step of recovering the variant polypeptide having DON degradation activity of the present disclosure, which is expressed in the culturing step.

In another embodiment, the variant polypeptide having DON degradation activity which is expressed in the culturing step may be recovered using methods known in the art to which the present disclosure pertains. For example, the variant polypeptide may be recovered from a medium by common procedures comprising, but not limited to, collection, centrifugation, filtration, extraction, spray-drying, evaporation, or precipitation.

The recovering method may be collecting the polypeptide using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, sonication, ultrafiltration, dialysis, various kinds of chromatographies such as molecular sieve chromatography (gel filtration), adsorption chromatography, ion exchange chromatography, affinity chromatography, etc., HPLC and a combination thereof may be used, and the variant polypeptide of the present disclosure may be recovered from the medium or the host cell using suitable methods known in the art.

In another embodiment, the variant polypeptide expressed by the host cell in the culturing step may not be recovered. In the embodiment, the host cell itself expressing the variant polypeptide may be used as a source of the variant polypeptide.

Still another aspect of the present disclosure provides use of any one or more of the variant polypeptide of the present disclosure; and the microorganism expressing the variant polypeptide in degrading deoxynivalenol (DON).

The definitions of the terms are as described above.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1: Preparation of GhSPG-M1 variants

### Example 1-1. Preparation of GhM1

A polynucleotide (SEQ ID NO: 2) encoding a variant (hereinafter, referred to as GhM1; SEQ ID NO: 1) into which 31 mutations were introduced based on a *Gossypium harknessii*-derived hypothetical protein sequence (SEQ ID NO: 37) was synthesized by Cosmo genetech, and cloned into a pET vector (Novagen) to prepare an expression vector.

### Example 1-2. Preparation of GhM1 point mutation and combination variants

To improve the activity of GhM1 described in Example 1-1, mutation sites were selected and primers were designed to prepare 9 types of point mutation and combination variants (hereinafter, referred to as GhM2, GhM3, GhM4, GhM5, GhM6, GhM7, GhM8, GhM9, GhM10, SEQ ID NOS: 3, 5, 7, 9, 11, 13, 15, 17, 19). The mutation sites based on the amino acid sequence of SEQ ID NO: 1, post-mutation amino acids, and primer sequences for preparing the variants are listed in Table 1 below.

**[Table 1]**

| Nam e of varia nt | Muta tion | | SEQ ID NO. | Primer sequence (5' → 3') |
|---|---|---|---|---|
| GhM 2 | D62 E | Forw ard | 21 | AGAATTGACGTCCCGGAaATGAAGATGACGTTATATTTTATGGG |
| | | Reve rse | 22 | tTCCGGGACGTCAATTCTTTTCAAAAGGCTCATACCAAGCAC |
| GhM 3 | D62 Q | Forw ard | 23 | AGAATTGACGTCCCGcAgATGAAGATGACGTTATATTTTATGGG |
| | | Reve rse | 24 | cTgCGGGACGTCAATTCTTTTCAAAAGGCTCATACCAAGCAC |
| GhM 4 | V60I | Forw ard | 25 | TTTTGAAAAGAATTGACattCCGGATATGAAGATGACG |
| | | Reve rse | 26 | aatGTCAATTCTTTTCAAAAGGCTCATACCAAGCACG |
| GhM 5 | I83V | Forw ard | 27 | GCGCCAAGTGATCCAgtgGAGCGGACCATTTGGACATTTAGCAG |
| | | Reve rse | 28 | cacTGGATCACTTGGCGCACTACTTACATCTTCATATCCCAT |
| GhM 6 | R48 E | Forw ard | 29 | TTTGGACTTCTATTCGgaaGTGCTTGGTATGAGCCTTTTGAAAAG |
| | | Reve rse | 30 | ttcCGAATAGAAGTCCAAAGACACTTTAGGGTCTTTAATGCGAAAC |
| GhM 7 | T133 V | Forw ard | 31 | ATCACGGTCGATGACgtgTACAAGGCGTGTGAGAGATTCGAACGTC |
| | | Reve rse | 32 | cacGTCATCGACCGTGATGCCTATGTGCCCGAAACCGATTGG |
| GhM 8 | V60I/ V75L | Forw ard | 25 | TTTTGAAAAGAATTGACattCCGGATATGAAGATGACG |
| | | Reve rse | 26 | aatGTCAATTCTTTTCAAAAGGCTCATACCAAGCACG |
| | | Forw ard | 33 | TTATGGGATATGAAGATctgAGTAGTGCGCCAAGTGATCCAATAGAG |
| | | Reve rse | 34 | cagATCTTCATATCCCATAAAATATAACGTCATCTTCATATCCGGAAT |
| GhM | V60I/ | Forw | 25 | TTTTGAAAAGAATTGACattCCGGATATGAAGATGACG |
| 9 | A156 V | ard | | |
| | | Reve rse | 26 | aatGTCAATTCTTTTCAAAAGGCTCATACCAAGCACG |
| | | Forw ard | 35 | CGGATGACGGCTATGtGTTTATTAAAGACCCCGATGGCTACTG |
| | | Reve rse | 36 | CaCATAGCCGTCATCCGGCTTTTTCACAAATTCTACCCCAAG |
| GhM 10 | V60I/ V75L /A15 6V | Forw ard | 25 | TTTTGAAAAGAATTGACattCCGGATATGAAGATGACG |
| | | Reve rse | 26 | aatGTCAATTCTTTTCAAAAGGCTCATACCAAGCACG |
| | | Forw ard | 33 | TTATGGGATATGAAGATctgAGTAGTGCGCCAAGTGATCCAATAGAG |
| | | Reve rse | 34 | cagATCTTCATATCCCATAAAATATAACGTCATCTTCATATCCGGAAT |
| | | Forw ard | 35 | CGGATGACGGCTATGtGTTTATTAAAGACCCCGATGGCTACTG |
| | | Reve rse | 36 | CaCATAGCCGTCATCCGGCTTTTTCACAAATTCTACCCCAAG |

In detail, 9 types of GhM1 point mutation and combination variants were prepared by PCR using the polynucleotide encoding GhM1 (SEQ ID NO: 1) prepared in Example 1-1, which was cloned into the pET vector, as a template, primers (SEQ ID NOS: 21, 22, 23, 24, 25, 26, 27, 28, 29, 30, 31, 32, 33, 34, 35, and 36 in Table 1), and a PCR premix (iNtRON, cat no. 25185). PCR was performed using an Eppendorf Mastercycler Nexus GX2, and the reaction conditions are as follows:
Initial denaturation - 94°C, 2 min
Denaturation - 94°C, 20 sec
Annealing - 50°C, 10 sec
Extension - 72°C, 8 min (15 cycles from denaturation to extension)
Final Extension - 72°C, 5 min

The prepared truncated variants were ligated using a QuickChange Site-Directed Mutagenesis kit (Agilent, Cat# 200518), and then transformed into the *E. coli* Dh5α strain to identify the presence of sequence mutations through sequencing.

### Example 2: Evaluation of properties of GhM1 point mutation and combination variants

### Example 2-1. Comparative evaluation of activity of GhM1 point mutation and combination variants

The expression vectors of GhM1 and 9 types of variants thereof prepared in Example 1-1 were each transformed into *E. coli* BL21 (DE3), which was inoculated into a sterile LB medium (BD Difco), and pre-cultured at 37°C, 200 rpm for 16 hours. Thereafter, 1/100 of the medium volume was inoculated into a flask comprising sterile LB medium, and cultured at 37°C, 200 rpm until the absorbance (OD₆₀₀) was between 0.4 to 0.5, and then isopropyl β-D-1-thiogalactopyranoside (IPTG) was added to a final concentration of 1 mM, and cultured for an additional 16 hours, and then the cells were collected by centrifugation. 20 ml of a lysis buffer (50 mM Tris-HCl pH 8.0, 100 mM NaCl, 10 mM imidazole) was added to the collected cells, which were resuspended therein, and a crude enzyme solution was obtained through sonication and centrifugation. The crude enzyme solution was applied and adsorbed onto Ni-NTA resin (Qiagen, Cat no. 30230), and then the enzyme was purified by sequentially applying a washing buffer (only 20 mM imidazole in the lysis buffer composition) and an elution buffer (only 250 mM imidazole in the lysis buffer composition).

Protein concentrations were determined by mixing 5 µl of the diluted enzyme solution + 250 µl of Bradford solution (Quick Start^{™} Bradford 1x Dye Reagent, #5000205), and measuring the absorbance at 595 nm.

To analyze the activities of the purified GhM1 and GhM1 point mutation and combination variants for DON, DON (CAS 51481-10-8, Deoxynivalenol) dissolved in distilled water and the purified enzyme reaction solution (25 mM Tris HCl, 150 mM NaCl, pH 7.4) were prepared, and after treatment with 500 µM of NiCl2 as a co-factor, the reaction occurred at 50°C for 24 hours. The reaction was then stopped by leaving it at 100°C for 5 minutes. The residual amount of DON in the reaction product was measured using high-performance liquid chromatography-ultraviolet (HPLC-UV) detection, and the relative activities of the variants were measured using the ratio of the total degraded DON. The results are as shown in Table 2 and FIG. 1.

**[Table 2]**

| **Variant** | **Relative Activity (%)** |
|---|---|
| **GhM1** | 100.0 |
| **GhM2** | 109.0 |
| **GhM3** | 107.7 |
| **GhM4** | 126.9 |
| **GhM5** | 106.0 |
| **GhM6** | 113.0 |
| **GhM7** | 105.0 |
| **GhM8** | 134.5 |
| **GhM9** | 138.4 |
| **GhM10** | 142.4 |

As a result, it was confirmed that, when the enzyme activity was expressed as a relative ratio, the activities of the 9 types of GhM1 point mutation and combination variants (GhM2, GhM3, GhM4, GhM5, GhM6, GhM7, GhM8, GhM9, GhM10) improved by 5% to 42% or more, as compared to the template GhM1.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

### [Sequence Listing]

| SEQ ID No. | SEQ Name | Sequence(5'→3') |
|---|---|---|
| 1 | GhM1 AA | MASSGPKESAANNPGLQTPPDEATKGYILQQTMFRIKDPKVSLDFYSRVL GMSLLKRIDVPDMKMTLYFMGYEDVSSAPSDPIERTIWTFSRPATIELTHF WGTESDPEFKGYHNGNSEPIGFGHIGITVDDTYKACERFERLGVEFVKKP DDGYAFIKDPDGYWIEIFDLKNIRNVVNGVA |
| 2 | GhM1 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGCGCGT GCTTGGTATGAGCCTTTTGAAAAGAATTGACGTCCCGGATATGAAGATG ACGTTATATTTTATGGGATATGAAGATGTAAGTAGTGCGCCAAGTGATCC AATAGAGCGGACCATTTGGACATTTAGCAGACCAGCGACAATCGAATTG ACCCATTTTTGGGGTACCGAGAGCGACCCGGAGTTTAAAGGTTACCATA ATGGCAATTCTGAACCAATCGGTTTCGGGCACATAGGCATCACGGTCG ATGACACTTACAAGGCGTGTGAGAGATTCGAACGTCTTGGGGTAGAATT TGTGAAAAAGCCGGATGACGGCTATGCGTTTATTAAAGACCCCGATGGC TACTGGATAGAGATATTTGACTTGAAGAATATACGCAATGTGGTAAATGG AGTCGCTTGA |
| 3 | GhM2 AA | MASSGPKESAANNPGLQTPPDEATKGYILQQTMFRIKDPKVSLDFYSRVL GMSLLKRIDVPEMKMTLYFMGYEDVSSAPSDPIERTIWTFSRPATIELTHF WGTESDPEFKGYHNGNSEPIGFGHIGITVDDTYKACERFERLGVEFVKKP DDGYAFIKDPDGYWIEIFDLKNIRNVVNGVA |
| 4 | GhM2 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA |
| | | TGTTTCGCATTAAAGACCCTAAAGTGTCTTTGGACTTCTATTCGCGCGT GCTTGGTATGAGCCTTTTGAAAAGAATTGACGTCCCGGAaATGAAGATG ACGTTATATTTTATGGGATATGAAGATGTAAGTAGTGCGCCAAGTGATCC AATAGAGCGGACCATTTGGACATTTAGCAGACCAGCGACAATCGAATTG ACCCATTTTTGGGGTACCGAGAGCGACCCGGAGTTTAAAGGTTACCATA ATGGCAATTCTGAACCAATCGGTTTCGGGCACATAGGCATCACGGTCG ATGACACTTACAAGGCGTGTGAGAGATTCGAACGTCTTGGGGTAGAATT TGTGAAAAAGCCGGATGACGGCTATGCGTTTATTAAAGACCCCGATGGC TACTGGATAGAGATATTTGACTTGAAGAATATACGCAATGTGGTAAATGG AGTCGCTTGA |
| 5 | GhM3 AA | MASSGPKESAANNPGLQTPPDEATKGYILQQTMFRIKDPKVSLDFYSRVL GMSLLKRIDVPQMKMTLYFMGYEDVSSAPSDPIERTIWTFSRPATIELTHF WGTESDPEFKGYHNGNSEPIGFGHIGITVDDTYKACERFERLGVEFVKKP DDGYAFIKDPDGYWIEIFDLKNIRNVVNGVA |
| 6 | GhM3 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGCGCGT GCTTGGTATGAGCCTTTTGAAAAGAATTGACGTCCCGcagATGAAGATG ACGTTATATTTTATGGGATATGAAGATGTAAGTAGTGCGCCAAGTGATCC AATAGAGCGGACCATTTGGACATTTAGCAGACCAGCGACAATCGAATTG ACCCATTTTTGGGGTACCGAGAGCGACCCGGAGTTTAAAGGTTACCATA ATGGCAATTCTGAACCAATCGGTTTCGGGCACATAGGCATCACGGTCG ATGACACTTACAAGGCGTGTGAGAGATTCGAACGTCTTGGGGTAGAATT TGTGAAAAAGCCGGATGACGGCTATGCGTTTATTAAAGACCCCGATGGC TACTGGATAGAGATATTTGACTTGAAGAATATACGCAATGTGGTAAATGG |
| | | AGTCGCTTGA |
| 7 | GhM4 AA | MASSGPKESAANNPGLQTPPDEATKGYILQQTMFRIKDPKVSLDFYSRVL GMSLLKRIDIPDMKMTLYFMGYEDVSSAPSDPIERTIWTFSRPATIELTHFW GTESDPEFKGYHNGNSEPIGFGHIGITVDDTYKACERFERLGVEFVKKPD DGYAFIKDPDGYWIEIFDLKNIRNVVNGVA |
| 8 | GhM4 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGCGCGT GCTTGGTATGAGCCTTTTGAAAAGAATTGACattCCGGATATGAAGATGA CGTTATATTTTATGGGATATGAAGATGTAAGTAGTGCGCCAAGTGATCCA ATAGAGCGGACCATTTGGACATTTAGCAGACCAGCGACAATCGAATTGA CCCATTTTTGGGGTACCGAGAGCGACCCGGAGTTTAAAGGTTACCATAA TGGCAATTCTGAACCAATCGGTTTCGGGCACATAGGCATCACGGTCGA TGACACTTACAAGGCGTGTGAGAGATTCGAACGTCTTGGGGTAGAATTT GTGAAAAAGCCGGATGACGGCTATGCGTTTATTAAAGACCCCGATGGCT ACTGGATAGAGATATTTGACTTGAAGAATATACGCAATGTGGTAAATGGA GTCGCTTGA |
| 9 | GhM5 AA | MASSGPKESAANNPGLQTPPDEATKGYILQQTMFRIKDPKVSLDFYSRVL GMSLLKRIDVPDMKMTLYFMGYEDVSSAPSDPVERTIWTFSRPATIELTHF WGTESDPEFKGYHNGNSEPIGFGHIGITVDDTYKACERFERLGVEFVKKP DDGYAFIKDPDGYWIEIFDLKNIRNVVNGVA |
| 10 | GhM5 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGCGCGT GCTTGGTATGAGCCTTTTGAAAAGAATTGACGTCCCGGATATGAAGATG |
| | | ACGTTATATTTTATGGGATATGAAGATGTAAGTAGTGCGCCAAGTGATCC AgtgGAGCGGACCATTTGGACATTTAGCAGACCAGCGACAATCGAATTG ACCCATTTTTGGGGTACCGAGAGCGACCCGGAGTTTAAAGGTTACCATA ATGGCAATTCTGAACCAATCGGTTTCGGGCACATAGGCATCACGGTCG ATGACACTTACAAGGCGTGTGAGAGATTCGAACGTCTTGGGGTAGAATT TGTGAAAAAGCCGGATGACGGCTATGCGTTTATTAAAGACCCCGATGGC TACTGGATAGAGATATTTGACTTGAAGAATATACGCAATGTGGTAAATGG AGTCGCTTGA |
| 11 | GhM6 AA | MASSGPKESAANNPGLQTPPDEATKGYILQQTMFRIKDPKVSLDFYSEVL GMSLLKRIDVPDMKMTLYFMGYEDVSSAPSDPIERTIWTFSRPATIELTHF WGTESDPEFKGYHNGNSEPIGFGHIGITVDDTYKACERFERLGVEFVKKP DDGYAFIKDPDGYWIEIFDLKNIRNVVNGVA |
| 12 | GhM6 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGgaaGTG CTTGGTATGAGCCTTTTGAAAAGAATTGACGTCCCGGATATGAAGATGA CGTTATATTTTATGGGATATGAAGATGTAAGTAGTGCGCCAAGTGATCCA ATAGAGCGGACCATTTGGACATTTAGCAGACCAGCGACAATCGAATTGA CCCATTTTTGGGGTACCGAGAGCGACCCGGAGTTTAAAGGTTACCATAA TGGCAATTCTGAACCAATCGGTTTCGGGCACATAGGCATCACGGTCGA TGACACTTACAAGGCGTGTGAGAGATTCGAACGTCTTGGGGTAGAATTT GTGAAAAAGCCGGATGACGGCTATGCGTTTATTAAAGACCCCGATGGCT ACTGGATAGAGATATTTGACTTGAAGAATATACGCAATGTGGTAAATGGA GTCGCTTGA |
| 13 | GhM7 AA | MASSGPKESAANNPGLQTPPDEATKGYILQQTMFRIKDPKVSLDFYSRVL |
| | | GMSLLKRIDVPDMKMTLYFMGYEDVSSAPSDPIERTIWTFSRPATIELTHF WGTESDPEFKGYHNGNSEPIGFGHIGITVDDVYKACERFERLGVEFVKKP DDGYAFIKDPDGYWIEIFDLKNIRNVVNGVA |
| 14 | GhM7 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGCGCGT GCTTGGTATGAGCCTTTTGAAAAGAATTGACGTCCCGGATATGAAGATG ACGTTATATTTTATGGGATATGAAGATGTAAGTAGTGCGCCAAGTGATCC AATAGAGCGGACCATTTGGACATTTAGCAGACCAGCGACAATCGAATTG ACCCATTTTTGGGGTACCGAGAGCGACCCGGAGTTTAAAGGTTACCATA ATGGCAATTCTGAACCAATCGGTTTCGGGCACATAGGCATCACGGTCG ATGACgtgTACAAGGCGTGTGAGAGATTCGAACGTCTTGGGGTAGAATTT GTGAAAAAGCCGGATGACGGCTATGCGTTTATTAAAGACCCCGATGGCT ACTGGATAGAGATATTTGACTTGAAGAATATACGCAATGTGGTAAATGGA GTCGCTTGA |
| 15 | GhM8 AA | MASSGPKESAANNPGLQTPPDEATKGYILQQTMFRIKDPKVSLDFYSRVL GMSLLKRIDIPDMKMTLYFMGYEDLSSAPSDPIERTIWTFSRPATIELTHFW GTESDPEFKGYHNGNSEPIGFGHIGITVDDTYKACERFERLGVEFVKKPD DGYAFIKDPDGYWIEIFDLKNIRNVVNGVA |
| 16 | GhM8 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGCGCGT GCTTGGTATGAGCCTTTTGAAAAGAATTGACattCCGGATATGAAGATGA CGTTATATTTTATGGGATATGAAGATctgAGTAGTGCGCCAAGTGATCCAA TAGAGCGGACCATTTGGACATTTAGCAGACCAGCGACAATCGAATTGAC |
| | | CCATTTTTGGGGTACCGAGAGCGACCCGGAGTTTAAAGGTTACCATAAT GGCAATTCTGAACCAATCGGTTTCGGGCACATAGGCATCACGGTCGAT GACACTTACAAGGCGTGTGAGAGATTCGAACGTCTTGGGGTAGAATTT GTGAAAAAGCCGGATGACGGCTATGCGTTTATTAAAGACCCCGATGGCT ACTGGATAGAGATATTTGACTTGAAGAATATACGCAATGTGGTAAATGGA GTCGCTTGA |
| 17 | GhM9 AA | MASSGPKESAANNPGLQTPPDEATKGYILQQTMFRIKDPKVSLDFYSRVL GMSLLKRIDIPDMKMTLYFMGYEDVSSAPSDPIERTIWTFSRPATIELTHFW GTESDPEFKGYHNGNSEPIGFGHIGITVDDTYKACERFERLGVEFVKKPD DGYVFIKDPDGYWIEIFDLKNIRNVVNGVA |
| 18 | GhM9 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGCGCGT GCTTGGTATGAGCCTTTTGAAAAGAATTGACattCCGGATATGAAGATGA CGTTATATTTTATGGGATATGAAGATGTAAGTAGTGCGCCAAGTGATCCA ATAGAGCGGACCATTTGGACATTTAGCAGACCAGCGACAATCGAATTGA CCCATTTTTGGGGTACCGAGAGCGACCCGGAGTTTAAAGGTTACCATAA TGGCAATTCTGAACCAATCGGTTTCGGGCACATAGGCATCACGGTCGA TGACACTTACAAGGCGTGTGAGAGATTCGAACGTCTTGGGGTAGAATTT GTGAAAAAGCCGGATGACGGCTATgtgTTTATTAAAGACCCCGATGGCTA CTGGATAGAGATATTTGACTTGAAGAATATACGCAATGTGGTAAATGGAG TCGCTTGA |
| 19 | GhM1 0 AA | MASSGPKESAANNPGLQTPPDEATKGYILQQTMFRIKDPKVSLDFYSRVL GMSLLKRIDIPDMKMTLYFMGYEDLSSAPSDPIERTIWTFSRPATIELTHFW GTESDPEFKGYHNGNSEPIGFGHIGITVDDTYKACERFERLGVEFVKKPD |
| | | DGYVFIKDPDGYWIEIFDLKNIRNVVNGVA |
| 20 | GhM1 0 NT | ATGGCATCGTCCGGGCCGAAAGAGTCGGCTGCCAATAATCCTGGCTTG CAAACTCCACCTGACGAGGCCACTAAGGGTTATATTCTGCAACAGACAA TGTTTCGCATTAAAGACCCTAAAGTGTCTTTGGACTTCTATTCGCGCGT GCTTGGTATGAGCCTTTTGAAAAGAATTGACattCCGGATATGAAGATGA CGTTATATTTTATGGGATATGAAGATctgAGTAGTGCGCCAAGTGATCCAA TAGAGCGGACCATTTGGACATTTAGCAGACCAGCGACAATCGAATTGAC CCATTTTTGGGGTACCGAGAGCGACCCGGAGTTTAAAGGTTACCATAAT GGCAATTCTGAACCAATCGGTTTCGGGCACATAGGCATCACGGTCGAT GACACTTACAAGGCGTGTGAGAGATTCGAACGTCTTGGGGTAGAATTT GTGAAAAAGCCGGATGACGGCTATgtgTTTATTAAAGACCCCGATGGCTA CTGGATAGAGATATTTGACTTGAAGAATATACGCAATGTGGTAAATGGAG TCGCTTGA |
| 21 | GhM2-F primer | AGAATTGACGTCCCGGAaATGAAGATGACGTTATATTTTATGGG |
| 22 | GhM2-R primer | tTCCGGGACGTCAATTCTTTTCAAAAGGCTCATACCAAGCAC |
| 23 | GhM3-F primer | AGAATTGACGTCCCGcAgATGAAGATGACGTTATATTTTATGGG |
| 24 | GhM3-R primer | cTgCGGGACGTCAATTCTTTTCAAAAGGCTCATACCAAGCAC |
| 25 | GhM4-F primer | TTTTGAAAAGAATTGACattCCGGATATGAAGATGACG |
| 26 | GhM4-R primer | aatGTCAATTCTTTTCAAAAGGCTCATACCAAGCACG |
| 27 | GhM5-F primer | GCGCCAAGTGATCCAgtgGAGCGGACCATTTGGACATTTAGCAG |
| 28 | GhM5-R primer | cacTGGATCACTTGGCGCACTACTTACATCTTCATATCCCAT |
| 29 | GhM6-F primer | TTTGGACTTCTATTCGgaaGTGCTTGGTATGAGCCTTTTGAAAAG |
| 30 | GhM6-R primer | ttcCGAATAGAAGTCCAAAGACACTTTAGGGTCTTTAATGCGAAAC |
| 31 | GhM7-F primer | ATCACGGTCGATGACgtgTACAAGGCGTGTGAGAGATTCGAACGTC |
| 32 | GhM7-R primer | cacGTCATCGACCGTGATGCCTATGTGCCCGAAACCGATTGG |
| 33 | V75L-F primer | TTATGGGATATGAAGATctgAGTAGTGCGCCAAGTGATCCAATAGAG |
| 34 | V75L-R primer | cagATCTTCATATCCCATAAAATATAACGTCATCTTCATATCCGGAAT |
| 35 | A156V -F primer | CGGATGACGGCTATGtGTTTATTAAAGACCCCGATGGCTACTG |
| 36 | A156V -R primer | CaCATAGCCGTCATCCGGCTTTTTCACAAATTCTACCCCAAG |
| 37 | GhSP G-9 AA | MGSLDSKESPANNPGLHSPPDEATKGYILQQTMFRIKDPKPALEFYSRVL GMSLLNKVDVPYMKMTLYMMGYEDVSSAPSDPVEKTIWTFGRPATMELT HFWGTENDPEFKGYHDGNSEPTGFGHIGITVDDMYKACERFESLGVEFV KKPGDGYAFIKDPDGYWIEIFDLNGIRAIVNNLA |
| 38 | GhSP G-9 NT | ATGGGGTCCCTTGATAGCAAAGAATCGCCCGCTAATAACCCTGGACTTC ATAGTCCCCCCGACGAGGCCACAAAAGGCTACATCCTTCAACAAACTAT GTTCCGCATAAAAGACCCGAAGCCAGCACTGGAGTTCTATTCTAGAGTA TTGGGGATGTCATTACTGAACAAAGTGGATGTACCATACATGAAAATGA CCCTGTACATGATGGGTTACGAAGATGTTTCATCTGCCCCAAGCGATCC CGTCGAGAAGACTATTTGGACGTTCGGCCGTCCTGCAACGATGGAACT GACCCATTTTTGGGGGACCGAGAATGACCCCGAATTTAAGGGTTACCAT GACGGGAACTCAGAACCTACCGGTTTTGGCCATATTGGTATTACTGTGG ATGATATGTATAAAGCGTGCGAGCGGTTTGAGAGCCTTGGTGTTGAATT TGTTAAAAAACCAGGAGACGGATATGCTTTTATCAAGGACCCGGACGG |
| | | CTACTGGATTGAGATTTTTGATTTGAACGGAATACGGGCTATCGTAAATA ACTTAGCA |

## Claims

1. A variant polypeptide having deoxynivalenol (DON) degradation activity, wherein amino acids at positions corresponding to any one or more positions selected from positions 48, 60, 62, 83, and 133 of SEQ ID NO: 1 are substituted with different amino acids.

2. The variant polypeptide of claim 1, wherein the variant polypeptide further comprises a substitution of an amino acid corresponding to position 75 of SEQ ID NO: 1 with leucine; or a substitution of an amino acid corresponding to position 156 of SEQ ID NO: 1 with valine; or a combination thereof, in addition to the substitutions.

3. The variant polypeptide of claim 1, wherein the variant polypeptide comprises a substitution of the amino acid corresponding to position 48 of SEQ ID NO: 1 with glutamic acid; a substitution of the amino acid corresponding to position 60 of SEQ ID NO: 1 with isoleucine; a substitution of the amino acid corresponding to position 62 of SEQ ID NO: 1 with glutamic acid or glutamine; a substitution of the amino acid corresponding to position 83 of SEQ ID NO: 1 with valine; or a substitution of the amino acid corresponding to position 133 of SEQ ID NO: 1 with valine.

4. The variant polypeptide of claim 2, wherein the variant polypeptide comprises a substitution of the amino acid corresponding to position 60 of SEQ ID NO: 1 with isoleucine; and a substitution of the amino acid corresponding to position 75 of SEQ ID NO: 1 with leucine.

5. The variant polypeptide of claim 2, wherein the variant polypeptide comprises a substitution of the amino acid corresponding to position 60 of SEQ ID NO: 1 with isoleucine; and a substitution of the amino acid corresponding to position 156 of SEQ ID NO: 1 with valine.

6. The variant polypeptide of claim 2, wherein the variant polypeptide comprises a substitution of the amino acid corresponding to position 60 of SEQ ID NO: 1 with isoleucine; a substitution of the amino acid corresponding to position 75 of SEQ ID NO: 1 with leucine; and a substitution of the amino acid corresponding to position 156 of SEQ ID NO: 1 with valine.

7. The variant polypeptide of claim 1, wherein the variant polypeptide comprises any one selected from SEQ ID NO: 3, SEQ ID NO: 5, SEQ ID NO: 7, SEQ ID NO: 9, SEQ ID NO: 11, and SEQ ID NO: 13.

8. The variant polypeptide of claim 2, wherein the variant polypeptide comprises any one selected from SEQ ID NO: 15, SEQ ID NO: 17, and SEQ ID NO: 19.

9. The variant polypeptide of claim 1, wherein the variant polypeptide has improved DON degradation activity, as compared to a polypeptide consisting of the amino acid sequence of SEQ ID NO: 1.

10. A polynucleotide encoding the variant polypeptide of any one of claims 1 to 9.

11. A microorganism comprising any one or more of the variant polypeptide of any one of claims 1 to 9; and a polynucleotide encoding the variant polypeptide.

12. A composition for degrading deoxynivalenol (DON), the composition comprising any one or more of the variant polypeptide of any one of claims 1 to 9; and a microorganism expressing the variant polypeptide.

13. A feed additive composition comprising any one or more of the variant polypeptide of any one of claims 1 to 9; and a microorganism expressing the variant polypeptide.

14. A method of degrading deoxynivalenol (DON), the method comprising the step of reacting DON with any one or more of the variant polypeptide of any one of claims 1 to 9; and a microorganism expressing the variant polypeptide.

15. A method of preparing a feed product, the method comprising the step of mixing the feed additive composition of claim 13 with a feed component.

16. A method of preparing a variant polypeptide having DON degradation activity, the method comprising the steps of:
culturing the microorganism of claim 11; and
recovering the variant polypeptide having DON degradation activity of any one of claims 1 to 9, which is expressed in the culturing step.

17. Use of any one or more of the variant polypeptide of any one of claims 1 to 9; and a microorganism expressing the variant polypeptide in degrading deoxynivalenol (DON).
